# FASCICULE DE BREVET EUROPEEN

(11) **EP 1 478 316 B1**
(45) Date de publication et mention de la délivrance du brevet: **11.04.2007**
(21) Numéro de dépôt: 03717371.3
(22) Date de dépôt: 03.02.2003
(51) Int. Cl.: A61F 13/15

(54) **ARTICLE D'HYGIENE CORPORELLE ANTI-CHOC ET STERILE TEL QUE COUCHE CULOTTE**
STOSSDÄMPFENDER UND STERILER HYGIENEARTIKEL, Z.B. EINE WINDEL
SHOCK-ABSORBING AND STERILE BODY HYGIENE ARTICLE SUCH AS A PANTY-NAPKIN

(30) Priorité: 08.02.2002 FR 0201574
(43) Date de publication de la demande: 24.11.2004
(73) Titulaire: Jerou, Tahar, 28500 Vernouillet (FR)
(72) Inventeur: Jerou, Tahar, 28500 Vernouillet (FR)
(74) Mandataire: Santarelli
(86) Numéro de dépôt international: PCT/FR2003/000323
(87) Numéro de publication internationale: WO 2003/066164

(56) Documents cités:
- EP-A- 0 298 348
- EP-A- 0 916 328
- WO-A-93/21879
- WO-A-98/55065
- US-A- 3 563 242
- US-A- 3 587 579
- US-A- 3 645 264
- US-A- 4 886 509
- US-A- 5 495 856
- US-A- 5 571 079
- US-B1- 6 277 106

## Description

L'invention concerne un article d'hygiène corporelle, tel qu'une couche-culotte destinée à protéger l'appareil génital, par exemple après un accident ou une intervention chirurgicale ou même une simple irritation. Elle vise notamment, mais pas exclusivement, une couche-culotte pour garçons en bas âge venant d'être circoncis, ou pour des personnes accidentées, malades ou infirmes de sexe masculin ou féminin. Elle vise aussi bien une couche-culotte spécifiquement conçue dans ce but, qu'un article destiné, dans le même but, à coopérer avec une couche-culotte (ou culotte) classique. Elle vise enfin très généralement un article d'hygiène corporelle destiné à protéger une zone quelconque du corps de l'utilisateur/utilisatrice.

Le principe de construction des couches-culottes est bien connu. Ainsi que cela est rappelé notamment par les documents FR-A-2 413 048, FR-A-2 438 434 ou FR-A-2 607 366, une couche comporte principalement une feuille extérieure souple imperméable aux liquides, par exemple en polyéthylène, un matelas absorbant en une ou plusieurs couches à l'intérieur de cette couche souple, par exemple en ouate de cellulose, et le plus souvent une feuille intérieure perméable classiquement en matériau non-tissé.

Ainsi qu'on le sait, certaines traditions impliquent la circoncision des garçons, parfois à l'âge de quelques semaines ou de quelques mois, alors que l'enfant ne sait pas encore contrôler ses sphincters. L'utilisation d'une couche-culotte est alors mal appropriée tant que la cicatrisation n'est pas complète, puisqu'elle implique en pratique des frottements et des risques de coups en cas de mouvement sur la zone en cours de cicatrisation, de sorte qu'il faut souvent renoncer temporairement à toute couche-culotte, avec tous les désagréments qu'on peut imaginer.

Les mêmes problèmes peuvent apparaître après une intervention chirurgicale de quelque nature que ce soit sur l'appareil génital du bébé, voire d'un adulte incontinent, immobilisé ou infirme, homme ou femme.

Un premier objet de l'invention est ainsi une couche-culotte pouvant être utilisée alors que l'appareil génital doit être préservé vis-à-vis des chocs, voire vis-à-vis de simples contacts, mais aussi de l'urine de l'utilisateur/utilisatrice.

Un second objet de l'invention est un article destiné à coopérer avec une couche-culotte classique, voire avec une culotte classique, en sorte de préserver l'appareil génital vis-à-vis des chocs, voire vis-à-vis de simples contacts, ou encore de l'urine de l'utilisateur/utilisatrice.

En fait, d'autres zones du corps susceptibles de générer des fluides corporels peuvent mériter d'être protégées vis-à-vis de tout choc ou de tout frottement ; c'est notamment le cas de toute zone en cours de cicatrisation, ce que les pansements classiques ne sont pas bien capables de bien assurer.

C'est pourquoi encore un objet de l'invention est un article destiné à protéger une zone de corps vis-à-vis des chocs, voire vis-à-vis de simples contacts.

On comprend aisément que des articles plans, tels que ceux décrits par les documents EP-0 916 328 et EP-0 298 348 notamment, ne sauraient en soi assurer une telle fonction de protection.

L'invention propose à cet effet une couche-culotte adaptée à protéger l'appareil génital du porteur ou de la porteuse, comportant une feuille extérieure souple et imperméable et une couche absorbante disposée à l'intérieur de cette feuille extérieure, caractérisée en ce qu'elle comporte en outre une coquille de protection anti-choc perméable aux liquides, séparée de la feuille extérieure souple et imperméable par au moins une partie de la couche absorbante, et disposée et conformée d'une manière suffisamment rigide pour venir coiffer sans appui l'appareil génital.

Selon des dispositions préférées de l'invention, éventuellement combinées :
- la couche absorbante comporte deux couches respectivement disposées à l'extérieure et à l'intérieur de la coquille,
- la couche disposée à l'intérieur de la coquille est moins épaisse que la couche disposée à l'extérieur,
- les couches sont en contact par leurs bords avec une portion de couche absorbante destinée à passer sous le périnée et les fesses,
- la couche absorbante se prolonge sous le périnée et les fesses,
- la coquille est en une matière élastique ou plastique perforée d'une pluralité de trous,
- la coquille est en une matière tressée,
- la coquille est une matière textile encollée,
- une zone adhésive est ménagée sur sa surface intérieure pour fixation sur la peau du porteur,
- la coquille est longée intérieurement par une feuille perméable.

L'invention propose en outre un article hygiénique, destiné à coopérer avec une culotte ou une couche-culotte comportant une feuille extérieure souple et imperméable et une couche absorbante disposée à l'intérieur de cette feuille extérieure, comportant une coquille de protection anti-choc perméable aux liquides, munie de moyens de fixation propres à la positionner dans une configuration dans laquelle elle est séparée de la feuille extérieure souple et imperméable par au moins une partie de la couche absorbante, et disposée et conformée d'une manière suffisamment rigide pour venir coiffer sans appui l'appareil génital.

Selon des dispositions préférées de l'invention, selon ce second aspect :
- l'article comporte en outre une couche interne absorbante, longeant la face intérieure de la coquille perméable,
- l'article comporte en outre une feuille perméable longeant intérieurement la coquille perméable,
- les moyens de fixation sont conçus en sorte de fixer la coquille sur le corps,
- les moyens de fixation sont conçus en sorte de fixer la coquille à l'intérieur d'une culotte ou couche-culotte,
- la coquille est en une matière élastique ou plastique perforée d'une pluralité de trous.

Selon un autre aspect, l'invention propose un pansement adapté à protéger une zone du corps du porteur ou de la porteuse, comportant une feuille extérieure souple et imperméable et une couche absorbante disposée à l'intérieur de cette feuille extérieure, caractérisée en ce qu'elle comporte en outre une coquille de protection anti-choc perméable aux liquides, séparée de la feuille extérieure souple et imperméable par au moins une partie de la couche absorbante, et disposée et conformée d'une manière suffisamment rigide pour venir coiffer sans appui la zone à protéger.

On notera que la couche-culotte définie ci-dessus est un cas particulier d'un tel article.

Des objets, caractéristiques et avantages de l'invention ressortent de la description qui suit, donnée à titre d'exemple non limitatif, en regard des dessins annexés sur lesquels :
- la figure 1 est une vue en perspective d'une couche-culotte conforme à l'invention, la partie de protection de cette couche étant représentée en place et sous forme éclatée,
- la figure 2 en est une autre vue en perspective, qui diffère de celle de la figure 1 par le fait que la partie de protection est visible au travers de la couche extérieure de cette couche-culotte,
- la figure 3 est une vue partielle en coupe verticale de cette couche-culotte,
- la figure 4 est une vue partielle en coupe verticale d'une variante de la couche-culotte des figures 1 à 3, et
- la figure 5 est une vue en coupe partielle d'un article hygiénique pouvant coopérer avec une culotte ou une couche-culotte en sorte de former un ensemble équivalent à la couche de la figure 3.

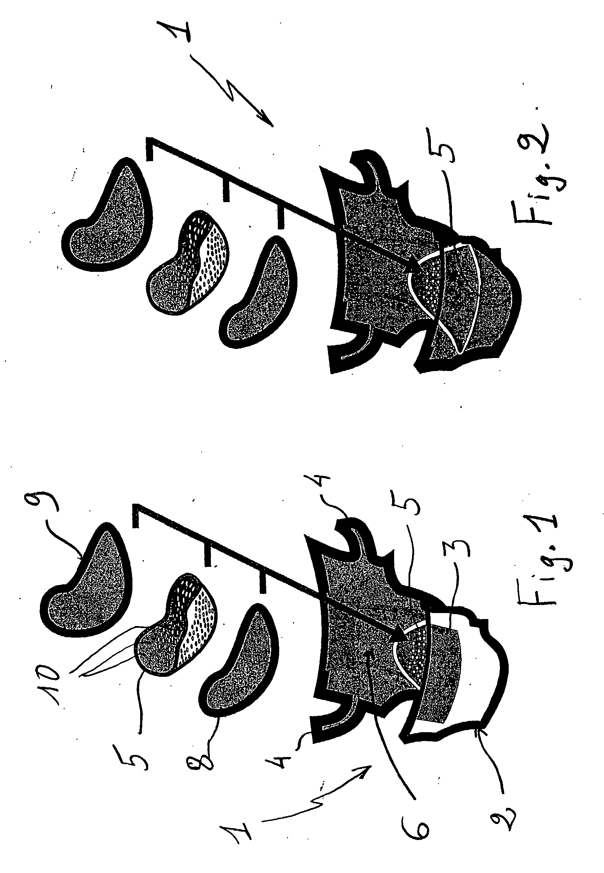

Les figures 1 à 3 représentent de manière générale un article d'hygiène destiné à protéger une zone du corps d'un porteur ou une porteuse, comportant une feuille extérieure souple et imperméable et une couche absorbante disposée à l'intérieur de cette feuille extérieure, caractérisée en ce qu'elle comporte en outre une coquille de protection anti-choc perméable aux liquides, séparée de la feuille extérieure souple et imperméable par au moins une partie de la couche absorbante, et disposée et conformée d'une manière suffisamment rigide pour venir coiffer sans appui la zone à protéger.

Plus précisément, dans l'exemple représenté, cet article est une couche-culotte 1 comportant, selon l'invention, une feuille extérieure souple et imperméable 2, munie classiquement de zones complémentaires de fixation 3 et 4, au moins une couche en matériau absorbant et une coquille rigide de protection 5 perméable aux liquides, qui est séparée de la feuille souple extérieure par au moins une partie de la couche en matériau absorbant et qui est destinée à venir coiffer l'appareil génital de l'utilisateur/utilisatrice de la couche-culotte 1.

En dehors de la zone comportant cette coquille en regard de l'appareil génital, la couche-culotte 1 a une configuration classique, avec par exemple une couche complémentaire de matériau absorbant 6 destinée notamment à passer sous le périnée et les fesses de l'utilisateur/utilisatrice, et des zones élastiques (non représentées) destinées au confort du port de la couche-culotte. Ces caractéristiques étant classiques, elles ne seront pas détaillées ici. En pratique la couche absorbante se raccorde à cette couche complémentaire 6 et, de manière préférée, une feuille souple perméable 7 (voir la figure 3) longe intérieurement la couche-culotte, au moins dans ses zones munies de matière absorbante.

Dans l'exemple représenté, la couche en matériau absorbant comporte deux couches 8 et 9 disposées respectivement à l'extérieur et à l'intérieur de la coquille 5.

La coquille 5 étant perméable, elle assure une possibilité d'écoulement de tout liquide depuis la couche intérieure 9 vers la couche 8, contribuant à minimiser l'humidité de la couche 9 la plus proche de l'appareil génital.

Pour favoriser un stockage du liquide à l'extérieur de la coquille, la couche extérieure 8 est de préférence plus épaisse que la couche intérieure 9. En variante non représentée, la couche 8 est omise, mais sa présence est avantageuse en ce qu'elle contribue au confort de l'utilisateur/utilisatrice, et permet une absorption rapide du liquide lorsqu'il apparaît.

La coquille perméable est destinée à protéger l'appareil génital vis-à-vis de contacts, pressions ou chocs intempestifs pendant le temps d'utilisation de la couche-culotte, que ce soit dans un lit (par exemple en cas de retournement du corps) ou en cas d'activités en principe calmes : cette coquille n'a donc pas besoin d'être aussi rigide, par exemple, que les coquilles utilisées pour protéger l'appareil génital lors de sports de combat ou brutaux. Cette coquille 5 est rigide en ce sens qu'elle présente une configuration concave adaptée à coiffer l'appareil génital masculin ou féminin de la personne considérée, et qu'elle peut conserver cette configuration pendant la durée d'utilisation d'une couche-culotte, c'est à dire à peine quelques heures.

Ainsi, cette coquille 5 peut être bien moins épaisse et résistante que les coquilles de sports. Elle est par exemple en matière plastique, polypropylène par exemple, ou en matière élastique telle que le caoutchouc, avec une épaisseur de quelques dixièmes de centimètres.

Dans l'exemple représenté, cette coquille 5 est en un matériau plastique imperméable, et est rendue perméable par la présence d'une pluralité de perforations 10, nombreuses et de préférence bien réparties sur toute la surface de la coquille.

Il est à noter que, compte tenu de la nature généralement filamentaire non tissée du matériau absorbant qui constitue la couche extérieure 8 et la couche intérieure 9 (par exemple de la ouate de cellulose), ces perforations peuvent servir à la mise en contact entre des filaments de chacune des couches, ce qui facilite le drainage du liquide depuis l'intérieur de la coquille vers l'extérieur.

En variante non représentée, cette coquille peut être formée d'un matériau textile, tissé ou non tissé, rigidifié par encollage en sorte de pouvoir conserver sa forme pendant plusieurs heures, sans que cette forme soit dégradée trop vite lorsqu'il est traversé par une décharge de liquide. Dans ce cas, la coquille est par construction perméable. D'autres configurations sont possibles ; c'est ainsi que la coquille peut aussi être réalisée par tressage de fils plastiques ou élastiques etc.... En fait la perméabilité de cette coquille, ainsi que cela vient d'être expliqué, peut provenir de la perméabilité intrinsèque du matériau qui la constitue, ou de la géométrie (par exemple, avec tressage, perforation, etc...) dans laquelle ce matériau est configuré.

Bien entendu, il est à la portée de l'homme de métier, en fonction de l'âge et du sexe du destinataire de la couche-culotte, de définir la géométrie (forme, et contour) de la coquille pour que sa présence ne nuise pas au confort en service.

Dans l'exemple représenté, les couches 8 et 9 prennent la coquille en sandwich et ont sensiblement le même contour (ces couches sont de préférence légèrement plus grandes que la coquille pour assurer que le liquide peut être assimilé sur toute la surface de cette coquille, y compris le long de ses bords). Le bord de ces couches (ou le bord de la couche extérieure si aucune couche intérieure est prévue) s'étend jusqu'au contact avec la couche 6 en sorte d'assurer une bonne continuité de pompage des liquides.

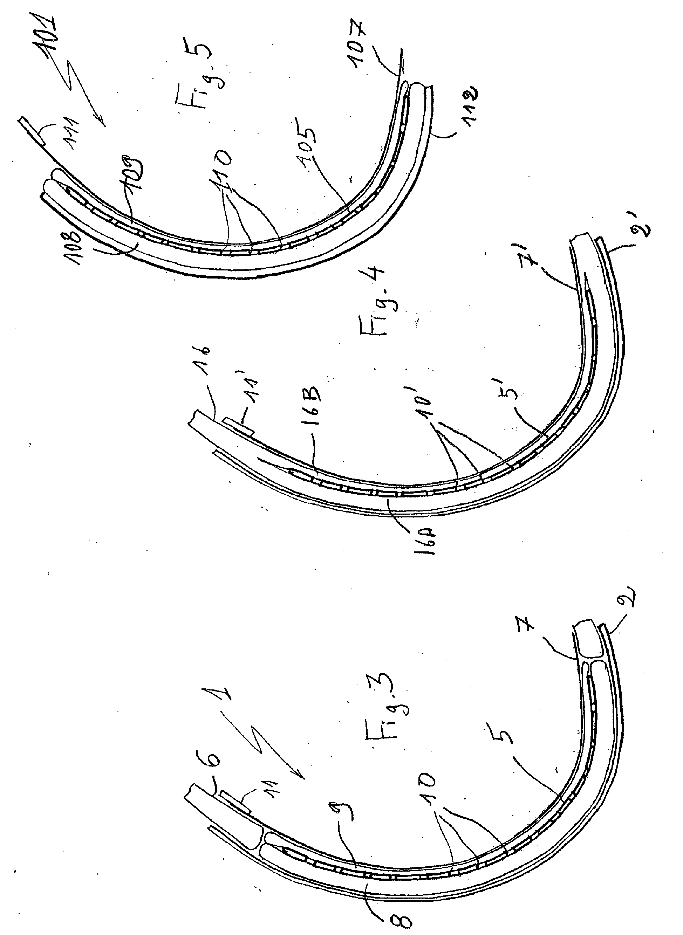

Dans la variante de réalisation de la figure 4, les couches extérieure et intérieure sont en fait le prolongement, en deux sous-couches 16A et 16B de la couche massive 16 qui est prévue ailleurs qu'en regard de l'appareil génital. La couche-culotte ne diffère de celle des figures 1 à 3 que sur ce point et ne sera pas décrite plus en détail. Sur cette figure les éléments analogues à ceux des figures 1 à 3 sont affectés de signes de référence qui découlent de ceux de ces figures 1 à 3 par adjonction de l'indice "prime".

La feuille souple perméable 7 ou 7', lorsqu'elle existe, est avantageusement collée à l'intérieur de la couche absorbante interne 9 ou 16B, voire directement sur la coquille. Elle est en regard de la zone de corps à protéger.

En pratique, les modalités classiques de fixation des couches-culottes sont suffisamment performantes pour assurer une bonne tenue en place. Toutefois, de manière préférée, pour garantir un bon maintien en place de la coquille, même en cas de mouvement, la couche-culotte de l'invention comporte avantageusement, sur sa surface intérieure, une zone adhésive 11 ou 11' permettant une adhésion temporaire au corps de l'utilisateur/utilisatrice, par exemple juste au-dessus de l'appareil génital.

La figure 5 représente un article permettant de constituer avec une couche-culotte classique (ou une culotte imperméable) classique non représentée, la configuration de la figure 3.

Sur cette figure 5, des éléments analogues à ceux de la figure 3 sont représentés par des chiffres de référence qui s'en déduisent par addition du nombre 100.

Cet article comporte ainsi une coquille de protection anti-choc 105 perméable aux liquides, munie de moyens de fixation à la peau 111 propres à positionner cette coquille sur une zone de corps d'un porteur ou porteuse dans une configuration dans laquelle elle peut venir coiffer sans appui cette zone.

Ainsi, lorsque cet article est porté avec une couche-culotte ou une culotte imperméable on retrouve une configuration dans laquelle cette coquille est séparée de la feuille extérieure souple et imperméable de cette culotte ou couche-culotte par une couche absorbante, faisant de préférence partie de cette culotte ou couche-culotte.

Toutefois, ainsi que cela est représenté cet article comporte avantageusement une couche absorbante 110 fixée à la face externe de la coquille pour bien assurer un drainage vers l'extérieur des fluides pouvant être générés à l'intérieur de la coquille.

De manière également préférée, cet article comporte une couche interne 109 d'absorbant longeant cette coquille améliorant l'absorption et le drainage de ces fluides.

Pour un bon confort, indépendamment de la présence de la couche 109, il y a avantageusement une feuille souple perméable 107 faisant face à la portion de corps à protéger.

Dans l'exemple représenté, les moyens de fixation 111 sont des zones d'adhésif destinées à être collées à la peau et fixées sur cette feuille 107 mais en variante non représentée, ils peuvent aussi être fixés sur une partie débordante de la couche externe d'absorbant, comme dans le cas d'un pansement classique, ce qui garantit la bonne tenue de la coquille. Ces moyens 111 de fixation à la peau peuvent aussi être fixés sur la couche interne 109 ou sur la coquille elle-même 105.

Dans la mesure où cet article est destiné à un usage unique, la tenue n'a pas nécessairement besoin d'être fixée avec la même qualité qu'un pansement.

En variante, cet article peut aussi être muni moyens de fixation indirecte au corps, c'est à dire de moyens de fixation à la culotte ou couche-culotte dans laquelle cet article est destiné à être porté, cette culotte ou couche-culotte étant munie de ses moyens spécifiques habituels de maintien sur le corps (c'est à dire les pattes adhésives classiques sur une couche culotte, ou la partie d'une culotte formant ceinture autour de la taille).

Ces moyens de fixation indirecte sont ici représentés sous la forme d'une couche d'adhésif 112, par exemple recouverte d'une feuille amovible de protection (non représentée) avant sa mise en service. Ces moyens 112 peuvent en variante être fixés sur toute autre partie de l'article (couche interne 109, feuille 107, voire la coquille 105 elle-même).

Les moyens 111 et 112 peuvent être combinés.

L'adhésif des moyens 111 est avantageusement choisi en sorte de ne pas créer d'allergie.

On comprend aisément que la configuration de la figure 3 ou celle de la figure 4 peuvent être d'usage très général, par exemple pour couvrir une zone de corps autre que l'appareil génital, la concavité de la coquille pouvant coiffer à distance, donc sans contact, une zone à préserver, par exemple une blessure en cours de cicatrisation et pouvant générer des fluides devant être évacués au mieux de cette blessure.

## Revendications

1. Article d'hygiène corporelle adapté à protéger une zone du corps du porteur ou de la porteuse, comportant une feuille extérieure souple et imperméable (2, 2') et une couche absorbante (8, 9, 16A, 16B) disposée à l'intérieur de cette feuille extérieure, **caractérisée en ce qu'**il comporte en outre une coquille de protection anti-choc (5, 5') perméable aux liquides, séparée de la feuille extérieure souple et imperméable par au moins une partie (8, 16A) de la couche absorbante, et disposée et conformée d'une manière suffisamment rigide pour venir coiffer sans appui la zone à protéger.

2. Article selon la revendication 1, formant une couche-culotte adaptée à protéger l'appareil génital du porteur ou de la porteuse.

3. Article selon la revendication 1 ou la revendication 2, **caractérisé en ce que** la couche absorbante comporte deux couches (8, 9, 16A, 16B) respectivement disposées à l'extérieure et à l'intérieur de la coquille.

4. Article selon la revendication 3, **caractérisé en ce que** la couche disposée à l'intérieur de la coquille est moins épaisse que la couche disposée à l'extérieur.

5. Article selon la revendication 3 ou la revendication 4, **caractérisé en ce que** les couches (8, 9) sont en contact par leurs bords avec une portion de couche absorbante (6) destinée à passer sous le périnée et les fesses.

6. Article selon l'une quelconque des revendications 1 à 4, **caractérisé en ce que** la couche absorbante (16A, 16B) se prolonge sous le périnée et les fesses.

7. Article selon l'une quelconque des revendications 1 à 6, **caractérisé en ce que** la coquille (5, 5') est en une matière élastique ou plastique perforée d'une pluralité de trous (10, 10').

8. Article selon l'une quelconque des revendications 1 à 6, **caractérisé en ce que** la coquille est en une matière tressée.

9. Article selon l'une quelconque des revendications 1 à 6, **caractérisé en ce que** la coquille est une matière textile encollée.

10. Article selon l'une quelconque des revendications 1 à 9, **caractérisée en ce qu'**une zone adhésive (11, 11') est ménagée sur sa surface intérieure pour fixation sur la peau du porteur.

11. Article selon l'une quelconque des revendications 1 à 10, **caractérisé en ce que** la coquille est longée intérieurement par une feuille perméable (7, 7').

12. Article hygiénique destiné à coopérer avec une culotte ou une couche-culotte comportant une feuille extérieure souple et imperméable et une couche absorbante disposée à l'intérieur de cette feuille extérieure, cet article comportant une coquille de protection anti-choc perméable aux liquides, munie de moyens de fixation (111, 112) propres à la positionner dans une configuration dans laquelle elle est séparée de la feuille extérieure souple et imperméable par au moins une partie de la couche absorbante, en coiffant l'appareil génital, cette coquille étant conformée d'une manière suffisamment rigide pour venir coiffer sans appui l'appareil génital.

13. Article hygiénique selon la revendication 12, **caractérisé en ce qu'**il comporte une couche d'absorbant (108) longeant extérieurement la coquille.

14. Article hygiénique selon la revendication 12 ou 13, **caractérisé en ce qu'**il comporte une couche d'absorbant (109) logeant intérieurement la coquille.

15. Article hygiénique selon l'une quelconque des revendications 12 à 14, **caractérisé en ce qu'**il comporte une feuille souple perméable en regard de l'appareil génital.

16. Article hygiénique selon l'une quelconque des revendications 12 à 15, **caractérisé en ce que** les moyens de fixation comportent des zones d'adhésif (111) destinées à coller à la peau.

17. Article hygiénique selon l'une quelconque des revendications 12 à 16, **caractérisé en ce que** les moyens de fixation comportent des zones d'adhésif (112) destinées à coller à la culotte ou couche-culotte.

18. Article selon l'une quelconque des revendications 1 à 11, **caractérisé en ce qu'**il est un pansement.

## Claims

1. Body hygiene article adapted to protect an area of the body of the male or female wearer, comprising an exterior flexible and impermeable film (2, 2') and an absorbent layer (8, 9, 16A, 16B) disposed inside this exterior film, **characterised in that** it further comprises an anti-impact protective shell (5, 5') permeable to liquids, separated from the exterior flexible and impermeable film by at least one portion (8, 16A) of the absorbent layer, and disposed and conformed in a sufficiently rigid manner to cap the area to be protected without pressing on it.

2. Article according to claim 1, forming a panty-napkin adapted to protect the genital system of the wearer.

3. Article according to claim 1 or claim 2, **characterised in that** the absorbent layer comprises two layers (8, 9, 16A, 16B) respectively outside and inside the shell.

4. Article according to claim 3, **characterised in that** the layer inside the shell is thinner than the layer outside it.

5. Article according to claim 3 or claim 4, **characterised in that** the layers (8, 9) are in contact at their edges with an absorbent layer portion (6) intended to pass under the perineum and the buttocks.

6. Article according to any of claims 1 to 4, **characterised in that** the absorbent layer (16A, 16B) extends under the perineum and the buttocks.

7. Article according to any of claims 1 to 6, **characterised in that** the shell (5, 5') is made from an elastic or perforated plastic material with a plurality of holes (10, 10').

8. Article according to any of claims 1 to 6, **characterised in that** the shell is made from a braided material.

9. Article according to any of claims 1 to 6, **characterised in that** the shell is made from a coated textile material.

10. Article according to any of claims 1 to 9, **characterised in that** an adhesive area (11, 11') is provided on its interior surface for fixing it to the skin of the wearer.

11. Article according to any of claims 1 to 10, **characterised in that** the shell is lined with a permeable film (7, 7').

12. Hygiene article adapted to co-operate with panties or a panty-napkin and comprising an exterior flexible and impermeable film and an absorbent layer disposed inside said exterior film, said article comprising an anti-impact protective shell permeable to liquids, provided with fixing means (111, 112) for positioning it in a configuration in which it is separated from the exterior flexible and impermeable film by at least one portion of the absorbent layer, capping the genital system, the shell being conformed in a sufficiently rigid manner to cap the genital system without pressing on it.

13. Hygiene article according to claim 12, **characterised in that** it comprises an absorbent layer (108) outside the shell.

14. Hygiene article according to claim 12, **characterised in that** it comprises an absorbent layer (109) inside the shell.

15. Hygiene article according to any of claims 12 to 14, **characterised in that** it comprises a flexible permeable film facing the genital system.

16. Hygiene article according to any of claims 12 to 15, **characterised in that** the fixing means comprise adhesive areas (111) adapted to stick to the skin.

17. Hygiene article according to any of claims 12 to 16, **characterised in that** the fixing means comprise adhesive areas (112) adapted to stick to the panties or panty-napkin.

18. Article accrding to any of claims 1 to 11, **characterised in that** it is a dressing.

## Patentansprüche

1. Körperhygieneartikel, der dazu vorgesehen ist, eine Zone des Körpers des Trägers oder der Trägerin zu schützen, umfassend eine weiche und undurchlässige äußere Folie (2, 2') und eine absorbierende Schicht (8, 9, 16A, 16B), die im Inneren dieser äußeren Folie angeordnet ist, **dadurch gekennzeichnet, dass** er ferner eine Stoßschutzschale (5, 5'), die für Flüssigkeiten durchlässig ist, umfasst, die von der weichen und undurchlässigen äußeren Folie durch mindestens einen Teil (8, 16A) der absorbierenden Schicht getrennt und derart angeordnet und ausreichend steif ausgeführt ist, dass sie sich an die zu schützende Zone anlegt, ohne auf ihr aufzuliegen.

2. Artikel nach Anspruch 1, der einen Hosenschutz bildet, der derart ausgeführt ist, dass er den Genitalbereich des Trägers oder der Trägerin schützt.

3. Artikel nach Anspruch 1 oder Anspruch 2, **dadurch gekennzeichnet, dass** die absorbierende Schicht zwei Schichten (8, 9, 16A, 16B) umfasst, die außerhalb bzw. innerhalb der Schale angeordnet sind.

4. Artikel nach Anspruch 3, **dadurch gekennzeichnet, dass** die im Inneren der Schale angeordnete Schicht weniger dick als die außen angeordnete Schicht ist.

5. Artikel nach Anspruch 3 oder Anspruch 4, **dadurch gekennzeichnet, dass** die Schichten (8, 9) mit ihren Rändern mit einem Abschnitt der absorbierenden Schicht (6) in Kontakt sind, der dazu bestimmt ist, unter dem Damm und dem Gesäß angeordnet zu werden.

6. Artikel nach einem der Ansprüche 1 bis 4, **dadurch gekennzeichnet, dass** sich die absorbierende Schicht (16A, 16B) unter dem Damm und dem Gesäß verlängert.

7. Artikel nach einem der Ansprüche 1 bis 6, **dadurch gekennzeichnet, dass** die Schale (5, 5') aus einem elastischen oder plastischen Stoff ist, der mit einer Vielzahl von Löchern (10, 10') perforiert ist.

8. Artikel nach einem der Ansprüche 1 bis 6, **dadurch gekennzeichnet, dass** die Schale aus einem geflochtenen Material ist.

9. Artikel nach einem der Ansprüche 1 bis 6, **dadurch gekennzeichnet, dass** die Schale aus einem geschlichteten Textilmaterial ist.

10. Artikel nach einem der Ansprüche 1 bis 9, **dadurch gekennzeichnet, dass** eine Haftzone (11, 11') auf seiner Innenseite zur Befestigung auf der Haut des Trägers vorgesehen ist.

11. Artikel nach einem der Ansprüche 1 bis 10, **dadurch gekennzeichnet, dass** die Schale innen mit einer durchlässigen Folie (7, 7') versehen ist.

12. Hygieneartikel, der dazu bestimmt ist, mit einer Hose oder einem Hosenschutz zusammenzuwirken, umfassend eine weiche und undurchlässige äußere Folie und eine absorbierende Schicht, die im Inneren dieser äußeren Folie angeordnet ist, wobei dieser Artikel eine Stoßschutzschale umfasst, die für Flüssigkeiten durchlässig und mit Befestigungsmitteln (111, 112) versehen ist, die sie in einer Konfiguration positionieren können, in der sie von der äußeren weichen und undurchlässigen Folie durch mindestens einen Teil der absorbierenden Schicht getrennt ist, wobei sie sich an den Genitalbereich anlegt, wobei diese Schale ausreichend steif ausgeführt ist, um sich an den Genitalbereich anzulegen, ohne auf ihm aufzuliegen.

13. Hygieneartikel nach Anspruch 12, **dadurch gekennzeichnet, dass** er eine absorbierende Schicht (108) umfasst, die außen auf der Schale aufgebracht ist.

14. Hygieneartikel nach Anspruch 12 oder 13, **dadurch gekennzeichnet, dass** er eine absorbierende Schicht (109) umfasst, die innen auf der Schale aufgebracht ist.

15. Hygieneartikel nach einem der Ansprüche 12 bis 14, **dadurch gekennzeichnet, dass** er eine weiche durchlässige Folie gegenüber dem Genitalbereich umfasst.

16. Hygieneartikel nach einem der Ansprüche 12 bis 15, **dadurch gekennzeichnet, dass** die Befestigungsmittel Haftzonen (111) umfassen, die dazu bestimmt sind, auf der Haut zu kleben.

17. Hygieneartikel nach einem der Ansprüche 12 bis 16, **dadurch gekennzeichnet, dass** die Befestigungsmittel Haftzonen (112) umfassen, die dazu bestimmt sind, auf der Hose oder dem Hosenschutz zu kleben.

18. Artikel nach einem der Ansprüche 1 bis 11, **dadurch gekennzeichnet, dass** es sich um einen Verband handelt.
